## Europäisches Patentamt
## European Patent Office
## Office européen des brevets

(19)

(11) Veröffentlichungsnummer: **0 066 806**
**B1**

(12)

# EUROPÄISCHE PATENTSCHRIFT

(45) Veröffentlichungstag der Patentschrift:
**11.07.84**

(51) Int. Cl.³: **C 09 B 7/10, C 07 D 333/64**

(21) Anmeldenummer: **82104698.4**

(22) Anmeldetag: **28.05.82**

(54) Verfahren zur Herstellung von 4,7-Dichlor-3-hydroxythionaphthen.

(30) Priorität: **03.06.81 DE 3121980**

(43) Veröffentlichungstag der Anmeldung:
**15.12.82 Patentblatt 82/50**

(45) Bekanntmachung des Hinweises auf die Patenterteilung:
**11.07.84 Patentblatt 84/28**

(84) Benannte Vertragsstaaten:
**CH DE FR LI**

(56) Entgegenhaltungen:
**CH - A - 168 447**
**DE - C - 966 698**
**FR - A - 2 293 473**
**US - A - 2 158 032**

(73) Patentinhaber: **HOECHST AKTIENGESELLSCHAFT,
Postfach 80 03 20, D-6230 Frankfurt am Main 80 (DE)**

(72) Erfinder: **Spietschka, Ernst, Dr., Kirchweg 3,
D-6270 Idstein/Taunus (DE)**
Erfinder: **Urban, Manfred, Steigerwaldstrasse 2A,
D-6200 Wiesbaden (DE)**

## Beschreibung

Aus der US-PS 2 158 032 ist ein Verfahren zur Herstellung von 4,7-Dichlor-3-hydroxythionaphthen durch aluminiumchloridkatalysierte Cyclisierung von 2,5-Dichlorphenyl-thioglykolsäurechlorid in Chlorbenzol bei 60°C bekannt. Das 4,7-Dichlor-3-hydroxythionaphthen wird dabei ohne Zwischenisolierung zum entsprechenden Tioindigo weiter oxidiert. Bei der Nacharbeitung dieses Verfahrens mit dem Ziele der Isolierung reinen 4,7-Dichlor-3-hydroxythionaphthens zeigte sich, dass Chlorbenzol unter diesen Bedingungen mit 2,5-Dichlorphenyl-thioglykolsäurechlorid reagiert und deshalb als Lösemittel für den gewünschten Zweck ausscheidet.

Auch andere Chlorkohlenwasserstoffe wie Tetrachlorethan, Ethylenchlorid, Perchlorethan, Tetrachlorkohlenstoff oder Fluorchlorkohlenwasserstoffe liefern 4,7-Dichlor-3-hydroxythionaphthen in nur unbefriedigender Reinheit. Ausserdem scheiden die meisten aliphatischen Chlorkohlenwasserstoffe wegen ihrer schlechten Regenerierbarkeit ohnehin aus. Auch wirken sich die beim Abdestillieren der Lösemittel teilweise notwendigen Temperaturen nachteilig auf die Reinheit des gebildeten temperaturempfindlichen 4,7-Dichlor-3-hydroxythionaphthens aus.

Überraschenderweise wurde nun gefunden, dass man bei Verwendung von Dichlormethan als Verdünnungsmittel bei der Cyclisierung von 2,5-Dichlorphenyl-thioglykolsäurechlorid in Gegenwart von Friedel-Crafts-Katalysatoren das 4,7-Dichlor-3-hydroxythionaphthen in der geforderten hohen Reinheit und sehr guter Ausbeute bei einer vollständigen Regenerierbarkeit des Dichlormethans erhält.

Die Erfindung betrifft deshalb ein Verfahren zur Herstellung von 4,7-Dichlor-3-hydroxythionaphthen durch Umsetzung von 2,5-Dichlorphenyl-thioglykolsäurechlorid in chlorierten Kohlenwasserstoffen in Gegenwart von Friedel-Crafts-Katalysatoren, das dadurch gekennzeichnet ist, dass die Umsetzung bei einer Temperatur von –20° bis +40°C, vorzugsweise 0° bis +30°C, insbesondere 0° bis 15°C, in Dichlormethan durchgeführt wird. Brauchbare Friedel-Crafts-Katalysatoren sind beispielsweise Aluminiumbromid und insbesondere Aluminiumchlorid.

In einer besonders bevorzugten Ausführungsform der Erfindung lässt man 2,5-Dichlorphenyl-thioglykolsäurechlorid bei 0° bis +15°C zu einer vorgelegten Suspension des Aluminiumhalogenids in Dichlormethan zutropfen. Zweckmässigerweise legt man 1,2 bis 1,4 Mol Aluminiumhalogenid je Mol 2,5-Dichlorphenyl-thioglykolsäurechlorid vor. Zweckmässigerweise wird zur Vervollständigung der Umsetzung unter guter Durchmischung bei etwa der gleichen Temperatur, die auch während des Zutropfens gewählt wurde, weitergerührt.

Nach der Zersetzung des Reaktionsgemisches mit Eis/Salzsäure wird das Dichlormethan zweckmässigerweise mittels Destillation abgetrennt und in den Prozess zurückgeführt. Das 4,7-Dichlor-3-hydroxythionaphthen wird abgesaugt und bei vermindertem Druck getrocknet.

Nach dem erfindungsgemässen Verfahren erhält man 4,7-Dichlor-3-hydroxythionaphthen in einer Reinheit, die für die Herstellung von 4,4',7,7'-Tetrachlorthioindigo bestens geeignet ist.

In den folgenden Beispielen beziehen sich Teile und Prozentangaben auf das Gewicht. 2,5-Dichlorphenyl-thioglykolsäurechlorid und 4,7-Dichlor-3-hydroxythionaphthen werden kurz mit A bzw. B bezeichnet.

Beispiel 1

Man liess 51,1 Teile A bei +10 bis +15°C innerhalb von 3 Stunden zu einer Suspension von 35 Teilen wasserfreiem Aluminiumchlorid in 100 Teilen wasserfreiem Dichlormethan zutropfen. Man rührte 2 Stunden bei +10 bis 15°C nach, goss das Reaktionsgemisch anschliessend auf eine Mischung aus 160 Teilen Eis, 40 Teilen Wasser, 4 Teilen konzentrierter Salzsäure und 0,4 Teilen einer 60%igen wässrigen Lösung eines Gemisches von sekundären Alkansulfonaten (C-Kettenverteilung: $<C_{13}$: $<1\%$, $C_{13}$–$C_{15}$: etwa 58%, $C_{16}$-$C_{17}$: etwa 39%, $>C_{17}$: $<3\%$) und rührte eine weitere halbe Stunde bei 0°C nach. Danach destillierte man das Dichlormethan bis zu einer Sumpftemperatur von 70°C ab, saugte ab, wusch neutral und trocknete bei 50°C und bei vermindertem Druck von etwa 200 mbar. Es wurden 44,5 Teile 96,1%iges B erhalten.

Beispiel 2

Man liess A bei 0°C zutropfen und rührte zur vollständigen Umsetzung bei 0°C nach. Ansonsten verfuhr man, wie im Beispiel 1 angegeben. Auf diese Weise wurden 44,6 Teile 97,1%iges B erhalten.

Beispiel 3

Man liess A bei +40°C zutropfen und rührte zur vollständigen Umsetzung bei +40°C nach. Ansonsten verfuhr man, wie im Beispiel 1 angegeben. Auf diese Weise wurden 43,7 Teile 93,2%iges B erhalten.

Beispiel 4

Man liess 51,1 Teile A bei +10 bis +15°C innerhalb einer Stunde zu einer Suspension von 35 Teilen wasserfreiem Aluminiumchlorid in 100 Teilen wasserfreiem Dichlormethan zutropfen. Man rührte eine Stunde bei +10 bis +15°C nach und goss das Reaktionsgemisch anschliessend auf die im Beispiel 1 beschriebene Mischung aus Eis, Wasser, Salzsäure und Alkansulfonat und verfuhr nun weiter, wie im Beispiel 1 angegeben. Auf diese Weise erhielt man 44,4 Teile 94,8%iges B.

Beispiel 5

1,1 Teile A wurden bei +10 bis +15°C innerhalb von 3 Stunden zu einer Suspension von 70 Teilen wasserfreiem Aluminiumbromid in 100 Teilen wasserfreiem Dichlormethan zutropfen gelassen. Man rührte 2 Stunden bei +10 bis +15°C nach und verfuhr weiter, wie im Beispiel 1 angegeben. Auf diese Weise erhielt man 42,7 Teile 89,5%iges B.

**Patentansprüche**

1. Verfahren zur Herstellung von 4,7-Dichlor-3-hydroxythionaphthen durch Umsetzung von 2,5-Dichlorphenyl-thioglykolsäurechlorid in chlorierten Kohlenwasserstoffen in Gegenwart von Friedel-Crafts-Katalysatoren, dadurch gekennzeichnet, dass die Umsetzung bei einer Temperatur von –20°C bis +40°C in Dichlormethan durchgeführt wird.

2. Verfahren nach Anspruch 1, dadurch gekennzeichnet, dass die Umsetzung bei einer Temperatur von 0° bis +30°C durchgeführt wird.

3. Verfahren nach Anspruch 1 und 2, dadurch gekennzeichnet, dass die Umsetzung bei einer Temperatur von 0° bis +15°C durchgeführt wird.

4. Verfahren nach Anspruch 1 bis 3, dadurch gekennzeichnet, dass man 2,5-Dichlorphenyl-thioglykolsäurechlorid zu einer vorgelegten Lösung oder Suspension des Friedel-Crafts-Katalysators in Dichlormethan zutropfen lässt und bis zur vollständigen Umsetzung unter guter Durchmischung bei etwa der gleichen Temperatur, die auch während des Zutropfens gewählt wurde, weiterrührt.

5. Verfahren nach Anspruch 1 bis 4, dadurch gekennzeichnet, dass man die Umsetzung in Gegenwart von Aluminiumchlorid durchführt.

6. Verfahren nach Anspruch 1 bis 5, dadurch gekennzeichnet, dass das Dichlormethan nach beendeter Reaktion abgetrennt und in den Prozess zurückgeführt wird.

7. 4,7-Dichlor-3-hydroxythionaphthen, erhalten nach einem der Verfahren gemäss Ansprüchen 1 bis 6.

**Claims**

1. A process for the preparation of 4,7-dichloro-3-hydroxythionaphthene by reaction of 2,5-dichlorophenyl-thioglycolic acid chloride in chlorinated hydrocarbons in the presence of Friedel-Crafts catalysts, characterized by carrying out the reaction at a temperature of from –20° to +40°C in dichloromethane.

2. The process according to claim 1, characterized by carrying out the reaction at a temperature of from 0° to 30°C.

3. The process according to claim 1 and 2, characterized by carrying out the reaction at a temperature of from 0° to 15°C.

4. The process according to claim 1 to 3, characterized by adding dropwise 2,5-dichlorophenyl-thioglycolic acid chloride to a solution or suspension of the Friedel-Crafts catalyst in dichloromethane, and continuing the intense intermixing of the batch by stirring at about the same temperature which was chosen for the dropwise addition.

5. The process according to claim 1 to 4, characterized by carrying out the reaction in the presence of aluminum chloride.

6. The process according to claim 1 to 5, characterized by separating the dichloromethane after completed reaction and recycling it to the process.

7. 4,7-dichloro-3-hydroxy-thionaphthene obtained according to one of the processes of claims 1 to 6.

**Revendications**

1. Procédé de préparation du dichloro-4,7 hydroxy-3 thionaphthène par réaction du chlorure de l'acide (dichloro-2,5 phényl)-thioglycolique, dans des hydrocarbures chlorés, en présence de catalyseurs de Friedel-Crafts, procédé caractérisé en ce qu'on effectue la réaction à une température de –20 à +40°C dans du dichlorométhane.

2. Procédé selon la revendication 1 caractérisé en ce qu'on effectue la réaction à une température de 0 à +30°C.

3. Procédé selon l'une des revendications 1 et 2, caractérisé en ce qu'on effectue la réaction à une température de 0 à +15°C.

4. Procédé selon l'une quelconque des revendications 1 à 3, caractérisé en ce qu'on ajoute goutte à goutte le chlorure de l'acide (dichloro-2,5 phényl)-thioglycolique à une solution ou suspension, préalablement mise en place, du catalyseur de Friedel-Crafts dans du dichlorométhane, et on continue d'agiter efficacement jusqu'à ce que la réaction soit complète, à peu près à la température à laquelle on a effectué l'addition goutte à goute.

5. Procédé selon l'une quelconque des revendications 1 à 4, caractérisé en ce que la réaction est effectuée en présence de chlorure d'aluminium.

6. Procédé selon l'une quelconque des revendications 1 à 5, caractérisé en ce que le dichlorométhane est séparé après la fin de la réaction et est renvoyé au procédé.

7. Dichloro-4,7 hydroxy-3 thionaphtène qui a été obtenu par un procédé selon l'une quelconque des revendications 1 à 6.